# EUROPÄISCHE PATENTSCHRIFT

(11) **EP 2 760 495 B1**
(45) Veröffentlichungstag und Bekanntmachung des Hinweises auf die Patenterteilung: **04.11.2015**
(21) Anmeldenummer: 12762004.5
(22) Anmeldetag: 24.09.2012
(51) Int. Cl.: A61M 1/00

(54) **OPHTHALMOCHIRURGISCHE KASSETTE**
OPHTHALMIC SURGICAL CASSETTE
CASSETTE DE CHIRURGIE OPHTALMOLOGIQUE

(30) Priorität: 28.09.2011 DE 102011114468
(43) Veröffentlichungstag der Anmeldung: 06.08.2014
(73) Patentinhaber: Carl Zeiss Meditec AG, 07745 Jena (DE)
(72) Erfinder: KÜBLER, Christoph, Oberkochen 73447 (DE); KRAUS, Martin, 73460 Hüttlingen (DE)
(74) Vertreter: Carl Zeiss AG - Patentabteilung
(86) Internationale Anmeldenummer: PCT/EP2012/068768
(87) Internationale Veröffentlichungsnummer: WO 2013/045395

(56) Entgegenhaltungen:
- EP-A1- 0 888 788
- US-A1- 2004 202 561
- US-A1- 2005 245 888

## Beschreibung

Die Erfindung betrifft eine ophthalmochirurgische Kassette und ein ophthalmochirurgisches System mit einer derartigen Kassette.

Zur Behandlung einer Linsentrübung, welche in der Medizin als grauer Star bezeichnet wird, gibt es mehrere chirurgische Techniken. Die am weitesten verbreitete Technik ist die Phakoemulsifikation, bei der eine dünne Spitze in eine erkrankte Linse eingeführt und mit Ultraschall zu Schwingungen angeregt wird. Die vibrierende Spitze emulsifiziert in ihrer nächsten Umgebung die Linse derart, dass die entstehenden Linsenfragmente durch eine Leitung von einer Pumpe abgesaugt werden können. Ist die Linse vollständig emulsifiziert worden, kann in den leeren Kapselsack eine neue künstliche Linse eingesetzt werden, so dass ein derart behandelter Patient wieder ein gutes Sehvermögen erreichen kann.

Bei der Phakoemulsifikation kommt ein System zum Einsatz, welches allgemein eine schwingfähige Spitze in einem Handstück, eine Spülleitung (Irrigationsleitung) für die Zufuhr von Spülfluid zu der zu behandelnden Linse und eine Saugleitung (Aspirationsleitung) zum Abtransportieren emulsifizierter Linsenfragmente einschließlich des Fluids in einen Sammelbehälter aufweist. Der Abtransport des Fluids und der Linsenfragmente kann mittels einer Peristaltikpumpe, auch Schlauchquetschpumpe genannt, erfolgen. Hierbei handelt es sich um eine Verdrängerpumpe, bei der ein Pumpenrad auf einen Schlauch drückt, in dem das Fluid enthalten ist. Durch eine Bewegung des Rades bei gleichzeitigem Druck auf den Schlauchmantel wird das Fluid innerhalb des Schlauches nach vorne transportiert. Der Vorteil einer solchen Pumpe liegt darin, dass das Fluid und die Linsenfragmente von den Pumpenkomponenten nicht direkt berührt werden, so dass keine Kontamination des Fluides durch die Pumpe erfolgen kann. Ferner können auch kleine Fördermengen relativ genau transportiert werden. Nachteilig ist jedoch, dass das Pumpenrad mit relativ hoher Kraft auf den Schlauch einwirken muss, so dass eine Notwendigkeit besteht, die zugehörige Rückhalteplatte oder Kassette mit großem Aufwand verwindungssteif zu konstruieren. Wenn ein Andrückkörper des Pumpenrades mit dem Schlauch in Eingriff kommt, entstehen innerhalb der Schlauchleitung im Fluid stoßartige Druckschwankungen oder Pulsationen. Da ein Pumpenrad mehrere Andrückkörper besitzt, werden pro Umdrehung eines Rades mehrere Drückstöße in das Fluid eingebracht. Bei einem kontinuierlichen Betrieb eines Pumpenrades entstehen somit zyklische Pulsationen innerhalb der Schlauchleitung. Da sich die Druckschwankungen des Fluids bis zum Auge fortpflanzen können, kann dies ab einer bestimmten Stärke der Druckschwankungen im Auge zu gefährlichen Verletzungen führen, welche unbedingt vermieden werden müssen.

In US 2008/0114312 A1 ist eine Saugpumpe beschrieben, welche aufweist: einen Pumpenkopf mit einer Vielzahl von Überständen, wobei der Pumpenkopf so angeordnet ist, dass er um eine Achse rotieren kann, eine Rampe, welche nahe der rotierenden Überstände angeordnet ist, wobei die Rampe einen zentralen Abschnitt, einen Eingangsabschnitt und einen Ausgangsabschnitt aufweist, wobei die Pumpe ferner einen elastischen Kanal aufweist, welcher konfiguriert ist, Fluid zu fördern, wenn er mit der Rampe und der Vielzahl von Überständen in Eingriff kommt.

Es ist somit eine Aufgabe, eine ophthalmochirurgische Kassette zu schaffen, welche im Zusammenwirken mit einer Peristaltikpumpe einen Abtransport von Fluid und Linsenfragmenten mit relativ niedriger Pulsationsfrequenz bei geringer Pulsationsstärke ermöglicht. Eine solche Kassette soll ferner in einer leichten und materialsparenden Konstruktion ausführbar sein. Ferner ist es eine Aufgabe, ein ophthalmochirurgisches System mit einer solchen Kassette zu schaffen.

Die Aufgabe wird für die ophthalmochirurgische Kassette durch einen Gegenstand mit den Merkmalen des unabhängigen Anspruchs 1 gelöst. Für das ophthalmochirurgische System wird die Aufgabe durch einen Gegenstand mit den Merkmalen des Anspruchs 10 gelöst. Vorteilhafte Weiterbildungen der Erfindung sind in den Unteransprüchen beschrieben.

Die erfindungsgemäße ophthalmochirurgische Kassette weist auf:
- einen Kassettenkörper, welcher an einer Schmalseite eine erste Aussparung aufweist, welche im Querschnitt in Form eines Kreissegmentes mit einem Kreisbogen ausgebildet ist, wobei die erste Aussparung so ausgebildet ist, dass ein Umfang eines Rades einer Peristaltikpumpe in die erste Aussparung eingreifen kann,
- eine Aspirationsleitung für den Transport eines mittels der Peristaltikpumpe aspirierten Fluides,
- wobei entlang des Kreisbogens des Kreissegmentes in radialer Richtung eine zweite Aussparung im Kassettenkörper vorgesehen ist, deren Wandung einen Fluidkanal bildet,
- wobei der Fluidkanal an einem ersten Ende mit der Aspirationsleitung verbunden ist und an einem zweiten Ende mit einer Sammelbehälterzuleitung verbunden ist,
- wobei die Kassette eine Haltevorrichtung zum Halten eines elastischen Abdeckelementes aufweist, mit dem der Fluidkanal abdeckbar ist, wobei die Haltevorrichtung einen konstanten Abstand des Abdeckelementes zur zweiten Aussparung sicherstellt, so dass mittels des auf das Abdeckelement einwirkenden Rades der Peristaltikpumpe Fluid von der Aspirationsleitung durch den abgedeckten Fluidkanal zur Sammelbehälterzuleitung transportierbar ist.

Die erfindungsgemäße Kassette weist an einer Schmalseite eine erste Aussparung auf, welche im Querschnitt in Form eines Kreissegmentes mit einem Kreisbogen ausgebildet ist, wobei in die erste Aussparung ein Umfang eines Rades einer Peristaltikpumpe eingreifen kann. Ein Pumpenrad wirkt somit senkrecht auf die Schmalseite ein, so dass ein hohes axiales Flächenträgheitsmoment des Kassettenkörpers genutzt werden kann. Dies bewirkt, dass die Durchbiegung des Kassettenkörpers nur einen sehr niedrigen Betrag einnimmt. Ein solcher Aufbau ist vorteilhaft im Vergleich zu Kassetten, bei denen das Pumpenrad auf eine Vorderseite der Kassette einwirkt, welche senkrecht zu den Schmalseiten der Kassette angeordnet ist. Bei einer solchen Krafteinwirkung auf die Vorderseite der Kassette tritt eine starke Durchbiegung der Kassette auf, so dass zur Verringerung dieses Effektes ein hoher Aufwand für eine verwindungssteife Konstruktion erforderlich ist. Indem die Schmalseite des Kassettenkörpers für den Eingriff des Pumpenrades genutzt wird, kann der Kassettenkörper nahezu ohne weitere Versteifungsrippen oder ähnliche Maßnahmen einfach und materialsparend konstruiert werden, wobei während des Betriebes der Pumpe und Eingreifen in die erste Aussparung der Kassette trotzdem eine hohe Verwindungssteifigkeit der Kassette vorliegt.

Die erfindungsgemäße Kassette ist ferner entlang des Kreisbogens des Kreissegmentes in radialer Richtung mit einer zweiten Aussparung versehen, deren Wandung einen Fluidkanal bildet, wobei die Kassette eine Haltevorrichtung zum Halten eines elastischen Abdeckelementes aufweist, mit dem der Fluidkanal abdeckbar ist. Es ist somit nicht erforderlich, wie im Stand der Technik üblich, das abzusaugende Fluid und die Linsenfragmente in einem Schlauch zu transportieren, welcher mit dem Pumpenrad in Eingriff kommt. Vielmehr besitzt die Kassette eine Haltevorrichtung, um ein elastisches Abdeckelement zu halten, mit dem der Fluidkanal abdeckbar ist. Das Pumpenrad wirkt somit nicht auf einen in die Kassette eingelegten Schlauch, sondern auf ein solches elastisches Abdeckelement ein. Indem die Haltevorrichtung so ausgebildet ist, dass sie einen konstanten Abstand des Abdeckelements entlang der zweiten Aussparung sicherstellt, transportiert das Pumpenrad das zu aspirierende Fluid immer zuverlässig entlang des Fluidkanals. Durch den konstanten Abstand des Abdeckelementes zur zweiten Aussparung hebt sich das Abdeckelement beim Eingreifen eines Pumpenrades nicht von der zweiten Aussparung ab, so dass ein konstanter Querschnitt des Fluidkanals und damit eine konstante Fluidförderrate erreicht werden.

Der konstante Abstand des Abdeckelementes zur zweiten Aussparung bewirkt ferner, dass es nicht wie im Stand der Technik erforderlich ist, relativ viele Andrückkörper eines Pumpenrades vorzusehen, um das Abheben eines Schlauches zu unterdrücken. Mit der erfindungsgemäßen Kassette ist es vielmehr möglich, mit nur wenigen Andrückkörpern eines Pumpenrades eine konstante Fluidförderrate zu erreichen. Dadurch werden auch nur relativ wenige Druckstöße in das zu fördernde Fluid induziert, so dass insgesamt eine geringere Pulsationsfrequenz in der Aspirationsleitung im Vergleich zu Kassetten gemäß dem Stand der Technik erreicht wird. Eine geringe Anzahl an Andrückkörpern führt noch zu einer weiteren vorteilhaften Wirkung: Je weniger Andrückkörper ein Pumpenrad besitzt, umso weniger Volumen des Fluidkanals wird von den Andrückkörpern beansprucht und umso mehr Fluid kann in einer vorbestimmten Länge des Fluidkanals transportiert werden. Damit lässt sich schon bei geringer Umdrehungszahl des Pumpenrades ein relativ hohes Fördervolumen erzielen. Dies ermöglicht eine zusätzliche Verringerung der Pulsationsfrequenz, wobei eine geringe Umdrehungszahl des Pumpenrades allgemein eine ruhigere Fluidförderung bei niedriger Pulsationsstärke bedeutet.

Vorzugsweise hält die Haltevorrichtung der Kassette das elastische Abdeckelement mittels Formschluss, Kraftschluss oder Stoffschluss. Die Haltevorrichtung kann die Geometrie des Abdeckelementes nutzen, um einen Formschluss zu erreichen. Die Haltevorrichtung kann zusätzlich oder alternativ auch so ausgebildet sein, dass es das Abdeckelement aufgrund eines Kraftschlusses mit dem Kassettenkörper hält, indem zwischen Abdeckelement und Kassettenkörper zum Beispiel eine Presspassung besteht. Wenn die Haltevorrichtung und das Abdeckelement zum Beispiel mittels Stoffschluss mit dem Kassettenkörper halten, kann die Haltevorrichtung zum Beispiel als ein Klebstoff ausgebildet sein.

Vorzugsweise ist die Haltevorrichtung ein vom Kassettenkörper abstehender Wulst oder eine in den Kassettenkörper eingebrachte Nut, so dass mindestens ein Formschluss erreicht wird. Besonders bevorzugt ist der Wulst oder die Nut auf einer Fläche senkrecht zur Schmalseite des Kassettenkörpers vorgesehen. Durch ein derartiges Umgreifen über Eck kann ein besonders sicherer Formschluss erzielt werden. Dass Abdeckelement kann auch einstückig mit der Kassette verbunden sein, zum Beispiel durch einen Zweikomponenten-Spritzguss.

Gemäß einer weiteren Ausführungsform variiert der Querschnitt des Fluidkanals in seiner Längsrichtung. In dem Bereich des Fluidkanals, in dem ein Andrückkörper des Pumpenrades erstmals mit dem Abdeckelement in Eingriff kommt, kann der Fluidkanal einen anderen Querschnitt besitzen als in dem sich daran anschließenden Bereich des Fluidkanals. Damit ist es möglich, die Stärke einer Pulsation des Aspirationsfluides deutlich zu verringern.

Vorzugsweise erstreckt sich der Fluidkanal an einem Ende tangential zu seiner Normalen an dem Ende fort. Der Fluidkanal kann sich an einem Ende auch als gekrümmte Bahn fort erstrecken, deren Krümmungsrichtung entgegengesetzt zur Krümmungsrichtung des Kreisbogens des Kreissegmentes der ersten Aussparung ist. Mittels eines tangentialen oder gekrümmten Einlauf- oder Auslaufbereiches des Fluidkanals kann ebenfalls die Pulsationsstärke verringert werden.

Gemäß einer weiteren Ausführungsform der Erfindung besitzt die Aspirationsleitung und/oder eine in die Kassette vorgesehene Irrigationsleitung eine starre Wandung aus dem gleichen Werkstoff wie der Kassettenkörper. Bei einer solchen Kassette ist es somit nicht erforderlich, für die Aspirationsleitung und/oder Irrigationsleitung einen Schlauch in die Kassette einzulegen. Vielmehr sind die Leitungen durch den Kassettenkörper gebildet. Damit lässt sich die Zeit verkürzen, in welcher eine erfindungsgemäße Kassette für eine Operation einsatzbereit gemacht werden kann.

Vorzugsweise ist die zweite Aussparung so vorgesehen, dass sich das Abdeckelement durch einen auf das Abdeckelement einwirkenden Andrückkörper des Rades der Peristaltikpumpe vollständig in die zweite Aussparung eindrücken lässt. Dies ermöglicht eine zuverlässige Abdichtung und einen sicheren Transport des Aspirationsfluids ohne Leckage-Verluste.

Vorzugsweise weist die Kassette zwei Hälften auf, welche sich zu dem Kassettenkörper zusammenfügen lassen. Damit lässt sich die Zeit für die Montage einer solchen Kassette deutlich reduzieren.

Das erfindungsgemäße ophthalmochirurgische System weist eine wie vorstehend beschriebene Kassette, eine Peristaltikpumpe, eine Steuerung zum Betrieb der Peristaltikpumpe und eine Eingabevorrichtung zum Eingeben von Signalen für die Steuerung auf.

Weitere Vorteile und Merkmale der Erfindung werden mit Bezug auf die nachfolgenden Zeichnungen erklärt, in welchen zeigen:
- Figur 1: eine isometrische Darstellung einer ersten Ausführungsform der ophthalmochirurgischen Kassette gemäß der Erfindung;
- Figur 2: eine Querschnittsansicht der ophthalmochirurgischen Kassette gemäß der Erfindung;
- Figur 3: eine Vorderansicht der erfindungsgemäßen Kassette mit einem montierten Abdeckelement;
- Figur 4: eine Querschnittsansicht eines Fluidkanals der erfindungsgemäßen Kassette mit einem montierten Abdeckelement;
- Figur 5a - 5c: Querschnittsansichten des Fluidkanals der erfindungsgemäßen Kassette mit einem montierten Abdeckelement, welches unterschiedlich stark in den Fluidkanal eingedrückt ist;
- Figur 6: eine schematische Darstellung der erfindungsgemäßen Kassette mit einem montierten Abdeckelement, in welches ein Pumpenrad einer Peristaltikpumpe eingreift;
- Figur 7: eine Querschnittsansicht einer zweiten Ausführungsform der erfindungsgemäßen Kassette mit tangential oder gekrümmt verlaufendem Einlauf- oder Auslaufbereich des Fluidkanals;
- Figur 8a-8b: Querschnittsansichten des Fluidkanals der erfindungsgemäßen Kassette mit unterschiedlichen Geometrien;
- Figur 9: eine schematische Darstellung einer erfindungsgemäßen Kassette mit einer darauf einwirkenden Peristaltikpumpe in einer Draufsicht;
- Figur 10: eine schematische Darstellung der Kräfteverhältnisse, welche auf die erfindungsgemäße Kassette beim Eingriff einer Peristaltikpumpe gemäß Figur 9 vorliegen;
- Figur 11: eine schematische Darstellung der erfindungsgemäßen Kassette mit darin enthaltenen Aspirations- und Irrigationsleitungen; und
- Figur 12: eine Darstellung des erfindungsgemäßen ophthalmochirurgischen Systems.

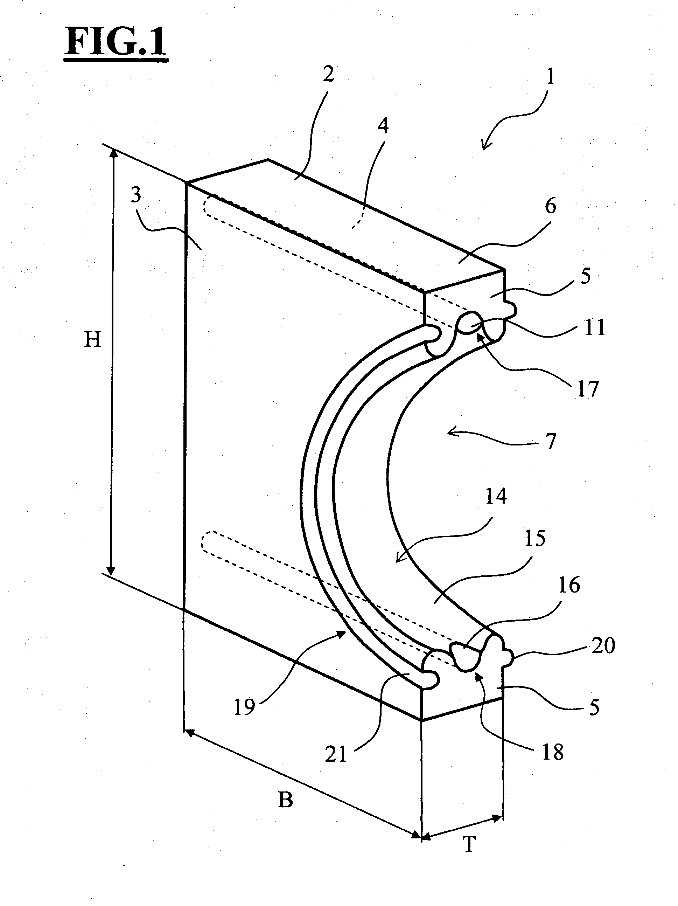

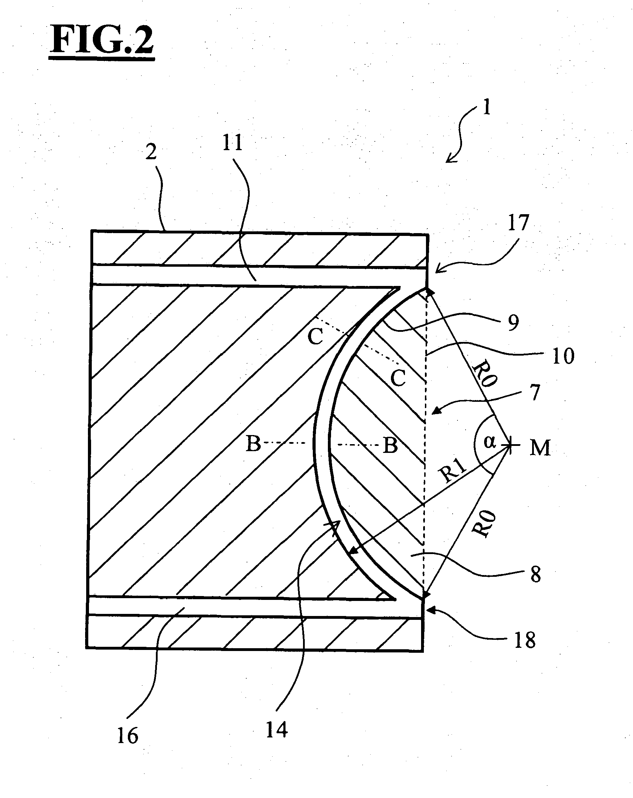

In Figur 1 und Figur 2 ist eine ophthalmochirurgische Kassette 1 gemäß der Erfindung dargestellt. Die Kassette 1 besitzt einen Kassettenkörper 2, welcher eine Vorderseite 3 und eine Rückseite 4 aufweist. Die Vorderseite 3 und Rückseite 4 besitzen jeweils eine Höhe H und Breite B. Der Kassettenkörper 2 besitzt eine Tiefe T, so dass die Rückseite 4 von der Vorderseite 3 in einem Abstand T zueinander angeordnet ist. Der so gebildete im Wesentlichen quaderförmige Kassettenkörper 2 besitzt damit eine rechte Schmalseite 5 und eine linke Schmalseiten 5, welche jeweils eine Seitenlänge mit dem Betrag T und dem Betrag H besitzen, und eine obere Schmalseite 6 und eine untere Schmalseite 6, welche jeweils eine Seitenlänge mit dem Betrag T und dem Betrag B besitzen.

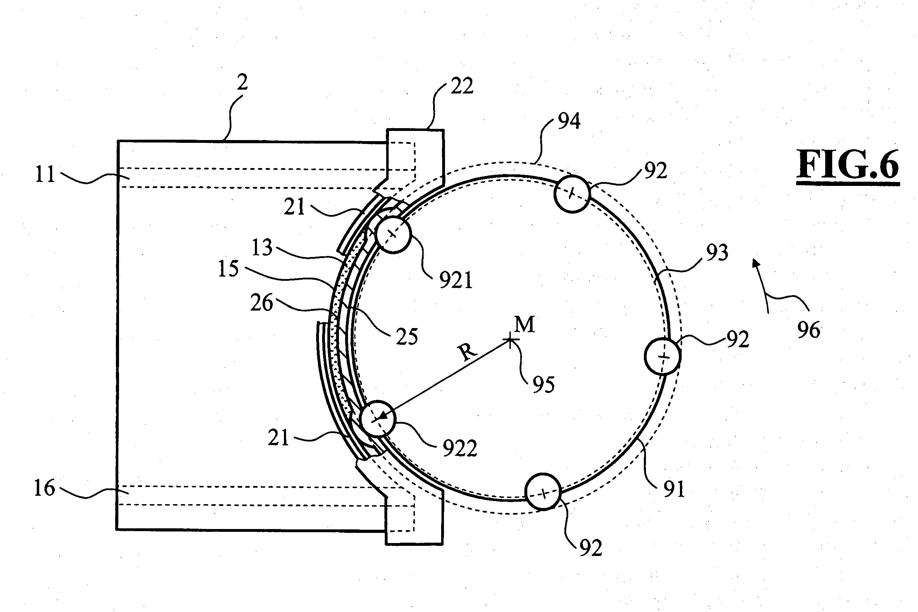

Bei der erfindungsgemäßen Kassette 1 weist eine erste Schmalseite 5 eine erste Aussparung 7 auf, welche im Querschnitt in Form eines Kreissegmentes 8 mit einem Kreisbogen 9 ausgebildet ist, siehe Figur 2. Der Kreisbogen 9 besitzt einen Radius R0 um einen Kreismittelpunkt M mit einem Mittelpunktswinkel a. Das Kreissegment 8 ist somit durch den Kreisbogen 9 und eine Kreissehne 10 begrenzt, wobei die Kreissehne 10 eine Basis eines gleichschenkligen Dreiecks mit den Schenkeln der Länge R0 bildet, welche sich im Kreismittelpunkt M schneiden. Die erste Aussparung 7 hat eine Größe, welche ausreicht, dass ein Umfang eines Rades 91 einer Peristaltikpumpe, siehe zum Beispiel Figur 6, in die erste Aussparung 7 eingreifen kann.

In dem Kassettenkörper 2 ist entlang des Kreisbogens 9 des Kreissegmentes 8 in radialer Richtung eine zweite Aussparung 14 vorgesehen, deren Wandung einen Fluidkanal 15 bildet. Die zweite Aussparung 14 besitzt, bezogen auf den Kreismittelpunkt M, einen Radius R1, welcher größer als der Radius R0 ist, siehe Figur 2.

Der Kassettenkörper 2 besitzt ferner eine Aspirationsleitung 11 für den Transport eines mittels der Peristaltikpumpe aspirierten Fluides. Die Aspirationsleitung 11 ist im Kassettenkörper 2 so angeordnet, dass sie mit dem Fluidkanal 15 an einem ersten Ende 17 so in Verbindung steht, dass Fluid von der Aspirationsleitung 11 in den Fluidkanal 15 strömen kann. Der Kassettenkörper 2 besitzt ferner eine Sammelbehälterzuleitung 16, welche mit dem Fluidkanal 15 an einem zweiten Ende 18 des Fluidkanals 15 verbunden ist, so dass aspiriertes Fluid vom Fluidkanal 15 über das zweite Ende 18 in die Sammelbehälterzuleitung 16 strömen kann.

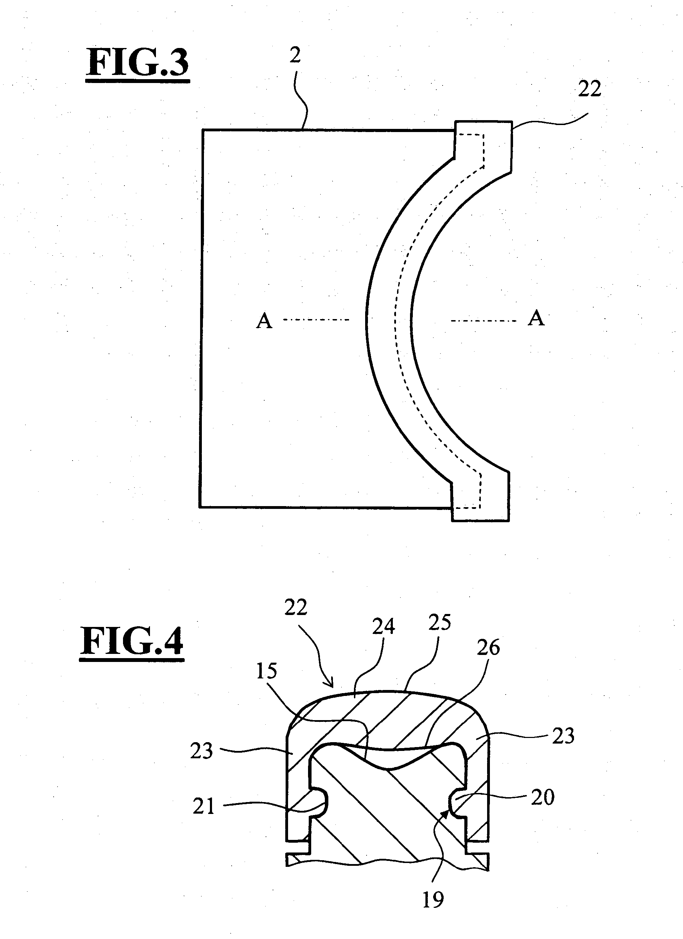

Die Kassette 1 weist ferner eine Haltevorrichtung 19 zum Halten eines elastischen Abdeckelementes 22 auf, siehe Fig. 3, mit dem der Fluidkanal 15 abdeckbar ist, wobei die Haltevorrichtung 19 einen konstanten Abstand des Abdeckelementes 22 entlang des Fluidkanals 15 sicherstellt. Wie aus Figur 1 ersichtlich ist, kann die Haltevorrichtung eine in den Kassettenkörper 2 eingebrachte Nut 21 sein. Die Haltevorrichtung 19 kann jedoch auch ein vom Kassettenkörper 2 abstehender Wulst 20 sein, siehe Figur 1. Die Haltevorrichtung 19 kann ferner auch in den Kassettenkörper 2 eingebrachte Bohrungen oder einen Klebstofffilm aufweisen.

Allgemein kann die Haltevorrichtung das elastische Abdeckelement 22 mittels Formschluss, Kraftschluss oder Stoffschluss mit dem Kassettenkörper 2 halten. Von Bedeutung ist dabei, dass die Haltevorrichtung 19 einen konstanten Abstand des Abdeckelementes 22 entlang des Fluidkanals 15 sicherstellt, wenn ein Andrückkörper eines Pumpenrades einer Peristaltikpumpe mit dem elastischen Abdeckelement 22 in Eingriff ist.

In Figur 3 ist eine Vorderansicht eines Kassettenkörpers 2 mit einem elastischen Abdeckelement 22 dargestellt. Das elastische Abdeckelement 22 ist im Wesentlichen bogenförmig ausgebildet. Figur 4 zeigt einen Querschnitt entlang der Schnittlinie A-A von Figur 3 des Kassettenkörpers 2 mit dem elastischen Abdeckelement 22. Das elastische Abdeckelement 22 besitzt einen im Wesentlichen U-förmigen Querschnitt mit zwei parallel zueinander angeordneten Seitenschenkeln 23 und eine diese verbindende Basis 24. Die Basis 24 besitzt eine konvexe obere Konturlinie 25 und eine konvexe untere Konturlinie 26. Zwischen der unteren Konturlinie 26 und dem Fluidkanal 15 des Kassettenkörpers 2 verbleibt ein Freiraum, in dem Fluid transportiert werden kann. Ein konstanter Abstand des Abdeckelementes 22 entlang des Fluidkanals 15 wird durch die Haltevorrichtung 19 derart sichergestellt, dass die beiden Seitenschenkel 23 des Abdeckelementes 22 einen nach außen vorstehenden Wulst besitzen, welche jeweils in eine Nut 21 des Kassettenkörpers 2 eingreifen. Damit ist eine formschlüssige Verbindung zwischen dem Abdeckelement 22 und dem Kassettenkörper 2 erreicht. Die Nut 21 kann ohne Unterbrechung äquidistant zum Fluidkanal 15 verlaufen. Es ist jedoch auch möglich, dass die Nut 21 Unterbrechungen aufweist, so dass äquidistant zum Fluidkanal 15 einzelne Nutabschnitte vorgesehen sind.

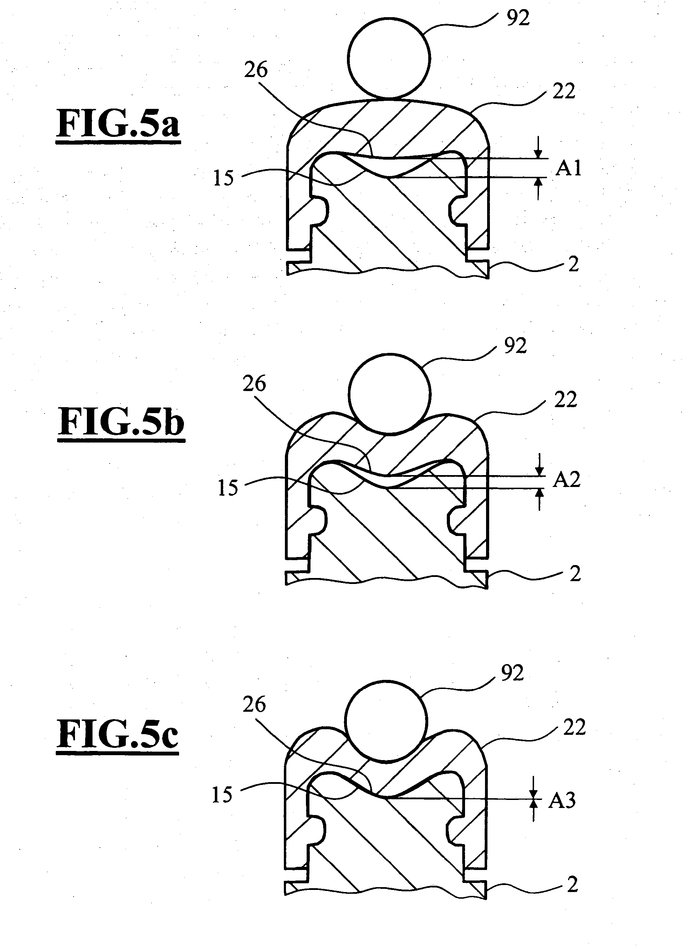

In den Figuren 5a bis 5c sind drei Stadien dargestellt, wie sich das elastische Abdeckelement 22 verformt, wenn ein Andrückkörper 92 eines Rades 91 einer Peristaltikpumpe auf das mit dem Kassettenkörper 2 montierte Abdeckelement 22 einwirkt. Figur 5a zeigt die Situation, bei der der Andrückkörper 92 mit dem elastischen Abdeckelement 22 erstmals in Kontakt kommt. Zwischen der unteren Konturlinie 26 und dem Fluidkanal 15 des Kassettenkörpers 2 ist ein noch maximal großer Abstand A1 vorhanden. Figur 5b zeigt die Situation, wenn der Andrückkörper 92 in das elastische Abdeckelement 22 eindrückt. Das Abdeckelement 22 wird teilweise in den Fluidkanal 15 eingedrückt, so dass nur noch ein geringerer Abstand A2 zwischen der unteren Konturlinie 26 des Abdeckelementes 22 und dem Fluidkanal 15 verbleibt. Figur 5c zeigt die Situation, bei der der Andrückkörper 92 das Abdeckelement 22 so stark nach unten drückt, dass der Fluidkanal 15 vollständig von der unteren Konturlinie 26 des Abdeckelementes 22 berührt wird. Der Abstand zwischen der unteren Konturlinie 26 und dem Fluidkanal 15 beträgt somit A3 = 0. An der Stelle, an der der Andrückkörper 92 auf das Abdeckelement wie bei Figur 5c eindrückt, ist somit der Fluidkanal 15 vollständig abgedichtet.

Figur 6 zeigt eine Vorderansicht des Kassettenkörpers 2 mit einem montierten Abdeckelement 22, wobei Andrückkörper 92 eines Pumpenrades 91 einer Peristaltikpumpe in das Abdeckelement 22 eingreifen. Die Mittelpunkte der Andrückkörper 92 sind auf einem Kreis 93 des Pumpenrades 91 angeordnet. Das Pumpenrad 91 besitzt eine Umfangslinie 94, welche die äußere Einhüllende der Andrückkörper 92 darstellt. Das Abdeckelement 22 ist so dimensioniert, dass ein in Drehrichtung 96 bewegender Andrückkörper 92 das Abdeckelement 22 gemäß der Schrittfolge in den Figuren 5a bis 5c zusammendrücken kann. Ein Andrückkörper 92 drückt somit das Abdeckelement 22 derart in den Fluidkanal 15, dass zwischen dem Abdeckelement 22 und dem Fluidkanal 15 kein Abstand mehr besteht, siehe auch Figur 5c. In Figur 6 ist diese Situation bei einem ersten Andrückkörper 921 dargestellt. Die gleiche Situation tritt bei einem benachbart dazu angeordneten Andrückkörper 922 auf. Dies bewirkt, dass zwischen dem ersten Andrückkörper 921 und dem zweiten Andrückkörper 922 im Fluidkanal 15 ein Fluid 13 gespeichert ist, welches bei fortgesetzter Bewegung des Pumpenrades 91 in der Antriebsrichtung 96 zur Sammelbehälterzuleitung 16 transportiert wird. Da der Kassettenkörper 2 bei dieser Ausführungsform mit einer Haltevorrichtung in Form einer Nut 21 versehen ist, welche sicherstellt, dass ein konstanter Abstand des Abdeckelementes 22 entlang des Fluidkanals 15 erreicht wird, ist die untere Konturlinie 26 des Abdeckelementes 22 im Bereich zwischen dem ersten Andrückkörper 921 und dem zweiten Andrückkörper 922 äquidistant zur tiefsten Linie des Fluidkanals 15. Zwischen dem ersten Andrückkörper 921 und dem zweiten Andrückkörper 922 hebt sich somit das Abdeckelement 22 nicht vom Fluidkanal 15 ab, so dass verhindert wird, dass das Abdeckelement 22 zwischen den beiden Andrückkörpern 921 und 922 in Form einer geradlinigen Strecke verläuft. Wie in Figur 6 gezeigt ist, verläuft stattdessen das Abdeckelement 22 zwischen den beiden Andrückkörpern 921 und 922 bogenförmig. Dies bewirkt, dass das Abdeckelement 22 zwischen den beiden Andrückkörpern 921 und 922 nicht gestreckt oder gestaucht wird, wodurch eine sehr gleichmäßige Transportbewegung und damit eine niedrige Pulsationsstärke des aspirierten Fluides erreicht wird.

In Figur 6 sind fünf Andrückkörper 92 eines Pumpenrades 91 einer Peristaltikpumpe dargestellt. Damit greifen pro Umdrehung eines Pumpenrades fünf Andrückkörper in das Abdeckelement 22 ein, so dass pro Umdrehung des Pumpenrades fünf mal eine geringfügige Pulsation oder Druckschwankung im Fluid 13 induziert wird. Die Pulsationshäufigkeit lässt sich verringern, wenn das Pumpenrad weniger als fünf Andrückkörper 92, zum Beispiel nur drei Andrückkörper 92, besitzt.

Bei Konstruktionen im Stand der Technik, bei denen anstatt eines Abdeckelementes 22 ein Schlauch vorgesehen ist, ist eine so geringe Anzahl an Andrückkörpern nicht sinnvoll. Ein Schlauch wird dabei zwischen dem ersten Andrückkörper 921 und dem zweiten Andrückkörper 922 geradlinig verlaufen und verbleibt nicht in einer bogenförmigen Kontur. Der Schlauch wird dabei gestreckt und gestaucht, so dass deutliche Druckschwankungen in das Fluid eingebracht werden. Die üblichen vielen Andrückkörper bewirken dann, dass der Schlauch mehr oder weniger bogenförmig in der Kassette liegen bleibt, so dass ein mehr oder weniger konstantes Fördervolumen erreicht wird. Die vielen Andrückkörper bewirken jedoch zusätzlich, dass das Schlauchmaterial sehr stark gestreckt wird, so dass deutliche Pulsationen im Druckverlauf des aspirierten Fluides erzeugt werden.

Die Hältevorrichtung des erfindungsgemäßen Kassette erlaubt nicht nur die Reduzierung von Andrückkörpern eines Pumpenrades, sondern ermöglicht auch den Betrieb eines Pumpenrades mit einem Durchmesser von zum Beispiel d = 200 mm, welches ein deutlich größerer Durchmesser im Vergleich zu Pumpenrädern gemäß dem Stand der Technik ist. Damit kann ein sehr großer Abstand zwischen zwei Andrückkörpern eines Pumpenrades erreicht werden, so dass sich eine sehr große Menge Fluid entlang des Fluidkanals mit nur wenigen induzierten Druckschwanlungen im Fluid transportieren lässt.

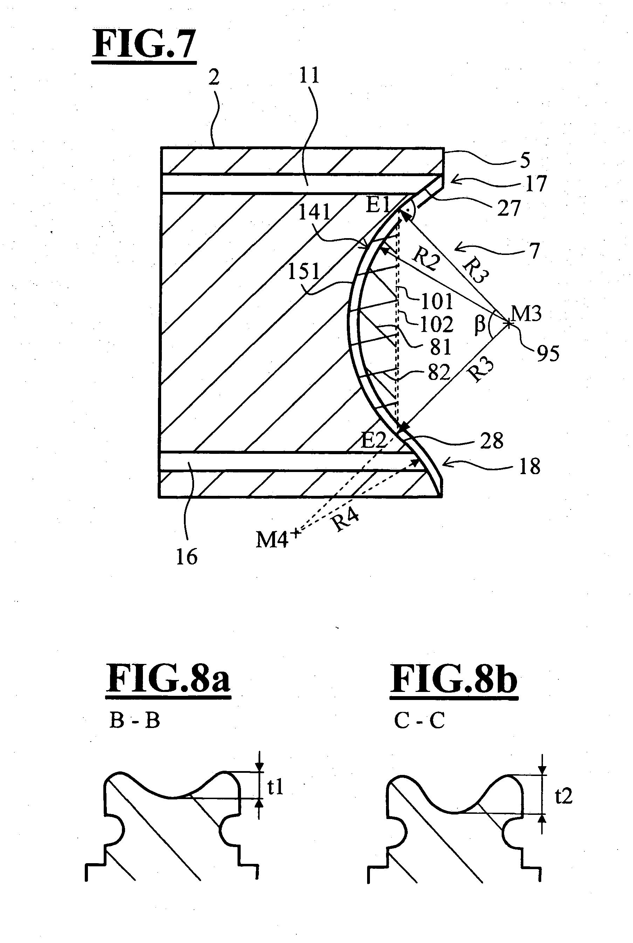

In Figur 7 ist ein Querschnitt einer weiteren Ausführungsform der erfindungsgemäßen Kassette dargestellt. Bei dieser Ausführungsform liegt eine Kreissehne 101 eines Kreissegmentes 81 mit einem Radius R2 innerhalb der ersten Aussparung 7 nicht in einer Linie mit der Seitenfläche einer Schmalseite 5 in Überdeckung, sondern ist in Richtung zum Inneren des Kassettenkörpers 2 hin verlagert. Entlang des Kreisbogens des Kreissegmentes 81 ist in radialer Richtung eine zweite Aussparung 141 vorgesehen, deren Wandung einen Fluidkanal 151 bildet. Wird die Kreissehne 101 entlang ihrer Längserstreckung zu beiden Seiten hin verlängert, bilden sich mit der tiefsten Linie des Fluidkanals 151 Schnittpunkte E1 und E2. Somit ergibt sich im Querschnitt ein Kreissegment 82 mit einem Kreisbogen entlang der tiefsten Linie des Fluidkanals 151 und einer Kreissehne 102, welche die Punkte E1 und E2 miteinander verbindet, wobei das Kreissegment 82 einen zugehörigen Mittelpunktswinkel β bei einem Radius R3 um M3 besitzt. Die Punkte E1 und E2 bilden somit jeweils ein Ende des Fluidkanals 151. Der Fluidkanal 151 kann nun bei dieser Ausführungsform vom Eckpunkt E1 an derart weiter verlaufen, dass er sich bezogen auf die Normale in E1 tangential von E1 fort erstreckt, siehe Tangente 27 in Fig. 7. Der Fluidkanal 151 besitzt somit eine bogenförmige Kontur und eine sich daran anschließende gerade verlaufende Kontur. Damit ist es möglich, dass ein sich auf einer Kreisbahn 93 verlaufender Andrückkörper 92 beim Eintreten in die erste Aussparung 7 mit zunehmender Bewegung in Bewegungsrichtung 96 zunehmend tiefer in das elastische Abdeckelement 22 eingreift. Ein solches zunehmendes Eingreifen bewirkt für das Fluid in dem Fluidkanal 151 eine noch niedrigere Druckschwankung und Pulsationsstärke als bei der ersten Ausführungsform gemäß Fig. 2.

Bei einer weiteren Ausführungsform erstreckt sich der Fluidkanal 151 an einem Ende E2 als gekrümmte Bahn fort, wobei deren Krümmungsrichtung entgegengesetzt zur Krümmungsrichtung des Kreisbogens des Kreissegmentes 81 der ersten Aussparung 7 ist. Der Fluidkanal 151 besitzt somit eine erste bogenförmige Kontur und eine sich daran anschließende zweite bogenförmige Kontur, welche jedoch entgegengesetzt zur ersten bogenförmigen Kontur gekrümmt ist. In Figur 7 ist dies am zweiten Ende 18 des Fluidkanals 151 dargestellt, wobei eine solche gekrümmte Bahn in gleicher Weise auch am ersten Ende 17 des Fluidkanals 151 vorgesehen sein könnte. Bei der in Figur 7 dargestellten Ausführungsform besitzt der Kreisbogen 28 einen Radius R4 um einen Mittelpunkt M4. Auch bei dieser Ausführungsform ist es möglich, dass ein sich auf einer Kreisbahn 93 verlaufender Andrückkörper 92 beim Eintreten in die erste Aussparung 7 mit zunehmender Drehbewegung entgegen der Bewegungsrichtung 96 tiefer in das elastische Abdeckelement 22 eingreift. Ein solches zunehmendes Eingreifen bewirkt für das Fluid in dem Fluidkanal 151 eine noch geringere Druckschwankung und Pulsationsstärke als bei der ersten Ausführungsform gemäß Fig. 2.

Die Pulsationsstärke des zu aspirierenden Fluids kann auch dadurch beeinflusst werden, indem der Querschnitt des Fluidkanals 15 oder 151 in seiner Längsrichtung variiert. In Figur 8a ist ein Querschnitt des Fluidkanals 15 entlang der Linie B-B von Figur 2 dargestellt. Der Fluidkanal 15 besitzt eine konkave Kontur mit einer Tiefe t1. Figur 8b zeigt einen Querschnitt eines Fluidkanals 15 entlang der Schnittlinie C-C von Figur 2, wobei der Fluidkanal eine konkave Kontur mit einer größeren Tiefe t2 besitzt.

Diese Variation des Querschnittes des Fluidkanals in seiner Längsrichtung ist nur ein Beispiel, wobei beliebige andere Geometrien möglich sind, zum Beispiel eine dreiecksförmige Kontur oder eine trapezförmige Kontur.

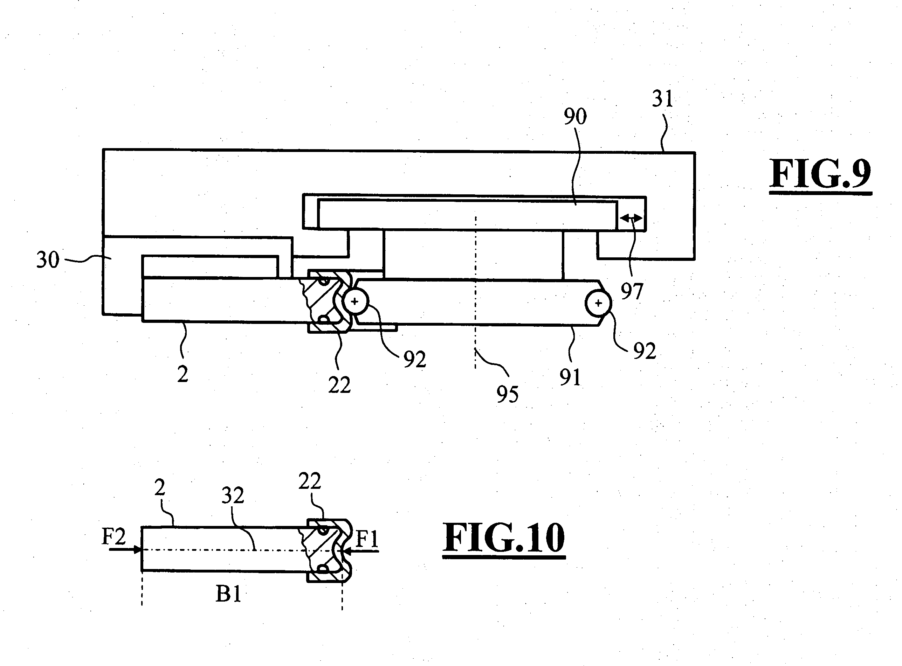

In Figur 9 ist eine Draufsicht der erfindungsgemäßen Kassette 1 dargestellt, wobei ein Pumpenrad 91 von einer Peristaltikpumpe 90 auf ein mit dem Kassettenkörper 2 montiertes Abdeckelement 22 einwirkt. Die Kassette 1 befindet sich in einer Kassettenaufnahme 30, welche mit einer Konsole 31 verbunden ist. Die Andrückkörper 92 des Pumpenrades 91 rotieren um eine Antriebsachse 95 der Peristaltikpumpe 90, wobei die Peristaltikpumpe 90 seitlich verschiebbar ist, siehe Doppelpfeil 97. Ein Kontakt zwischen einem der Andrückkörper 92 und dem Abdeckelement 22 erfolgt somit derart, dass nach dem Einsetzen der Kassette in die Kassettenaufnahme 30 die Peristaltikpumpe 90 seitlich auf das Abdeckelement hin bewegt wird. Entsprechend erfolgt ein Austausch der Kassette derart, dass zuerst die Peristaltikpumpe 90 von der Kassette nach rechts fort bewegt wird, bis sich die Abdeckkörper 92 und das Abdeckelement 22 nicht mehr berühren.

Figur 10 zeigt eine analog zur Figur 9 dargestellte Kassette 1, wobei auf die Kassette 1 wirkende Kraftvektoren F1 und F2 dargestellt sind. Wenn ein Andrückkörper 92 des Rades 91 der Peristaltikpumpe 90 auf das Abdeckelement 22 einwirkt, liegt der Kraftvektor F1 in einer Linie mit der Mittelebene 32 der Kassette 1. Die Kassettenaufnahme 30 setzt der einwirkenden Kraft F1 eine Reaktionskraft F2 entgegen, welche ebenfalls in einer Linie mit der Mittelebene 32 der Kassette liegt. Die Kräfte F1 und F2 wirken in einem Abstand B1 zueinander. Bei einer derartigen Kräfteverteilung ist das axiale Flächenträgheitsmoment des Kassettenkörpers relativ hoch, da bei der Berechnung des axialen Flächenträgheitsmomentes der Abstand B1 in der dritten Potenz eingeht.

Der Kassettenkörper 2 kann somit relativ einfach konstruiert sein, wobei kein größerer Aufwand für die Konstruktion von Versteifungsrippen oder ähnlichem erforderlich ist.

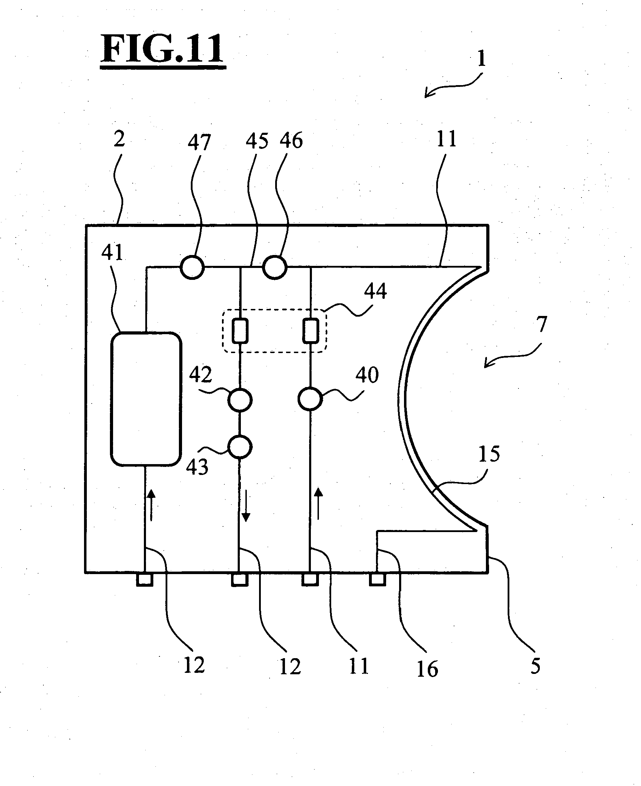

Figur 11 zeigt eine schematische Darstellung der erfindungsgemäßen Kassette 1. An der ersten Schmalseite 5 ist die erste Aussparung 7 vorgesehen, wobei innerhalb des Kassettenkörpers 2 mehrere Leitungen verlaufen. Der Kassettenkörper 2 weist eine Aspirationsleitung 11 und ein Schaltventil 40 auf, mit dem ein Fluiddurchfluss zuverlässig unterbrochen oder freigegeben werden kann. Die Aspirationsleitung 11 ist mit dem Fluidkanal 15 verbunden, an welchen sich die Sammelbehälterzuleitung 16 anschließt. Zusätzlich weist der Kassettenkörper 2 eine Irrigationsleitung 12 auf, mit welcher sich ein Irrigationsfluidbehälter 41 füllen lässt. Die Irrigationsleitung 12 wird dann zu einem Auslass des Kassettenkörpers 2 geführt, wobei eine Druckmessvorrichtung 42 für die Messung eines Druckes in der Irrigationsleitung 12 und eine Volumenstrommessvorrichtung 43 für eine Messung des Volumenstroms in der Irrigationsleitung 12 vorgesehen sind. Zusätzlich ist eine Differenzdruckmessvorrichtung 44 vorgesehen, mit welcher sich ein Differenzdruck zwischen der Aspirationsleitung 11 und der Irrigationsleitung 12 messen lässt. Die Irrigationsleitung 12 ist mittels einer Verbindungsleitung 45 und einem Refluxventil 46 mit der Aspirationsleitung 11 verbunden. Somit kann die Aspirationsleitung 11 durch Zufuhr von Irrigationsfluid befüllt werden, wenn das Refluxventil 46 geöffnet ist.

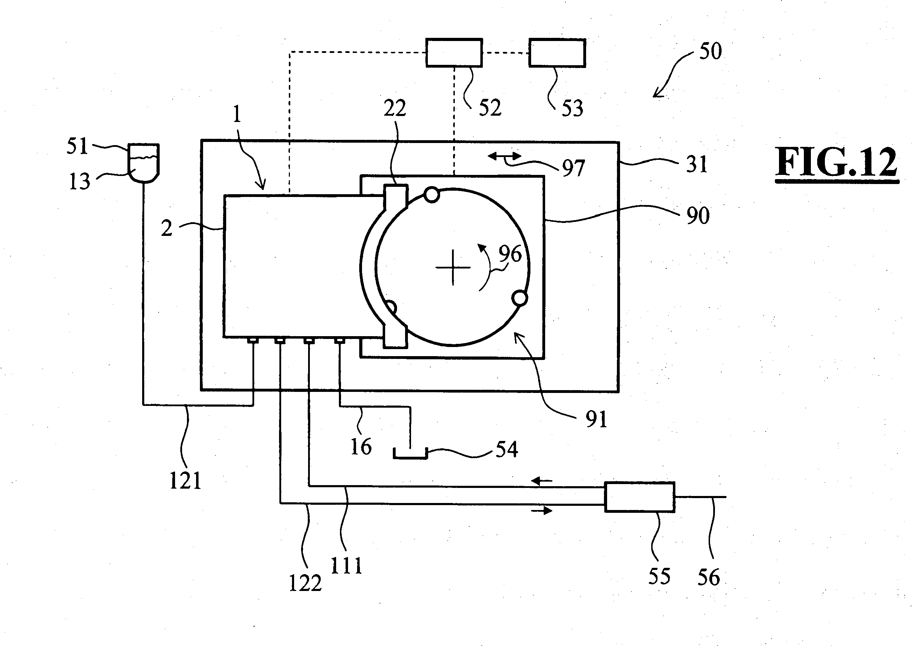

Figur 12 zeigt ein ophthalmochirurgisches System 50, welches eine Kassette 1, eine Konsole 31 und eine Peristaltikpumpe 90 mit einem Pumpenrad 91 aufweist. Das Pumpenrad 91 greift in die erste Aussparung 7 des Kassettenkörpers 2 ein. Eine Bewegung der Peristaltikpumpe 90 in Richtung zum Kassettenkörper 2 oder von diesem Kassettenkörper 2 fort gemäß dem Doppelpfeil 97 kann mittels einer Steuerungsvorrichtung 52 erfolgen, welche mit einer Eingabevorrichtung 53 gekoppelt ist. Die Steuerungsvorrichtung 52 ist ferner geeignet, die Drehrichtung des Pumpenrades 91, die Zeitdauer einer Drehung des Pumpenrades 91 und eine Drehgeschwindigkeit des Pumpenrades 91 einzustellen. In einem Irrigationsfluidbehälter 51 ist ein Irrigationsfluid 13 enthalten, welches mittels einer ersten Zufuhrleitung 121 zur Irrigationsleitung 12 im Kassettenkörper 2 gelangen kann. Das Irrigationsfluid verlässt die Irrigationsleitung 12 des Kassettenkörpers 2, um in eine zweite Zufuhrleitung 122 einzutreten, welche mit einem Phako-Handstück 55 verbunden ist. Abzusaugendes Fluid gelangt über eine dritte Zufuhrleitung 111 zur Aspirationsleitung 11 im Kassettenkörper 2. Bei einer Bewegung des Pumpenrades 91 in Antriebsrichtung 96 wird das Aspirationsfluid über den Fluidkanal 15 zur Sammelbehälterzuleitung 16 befördert, bis es aus dem Kassettenkörper 2 austritt und über die Sammelbehälterzuleitung 16 zu einem Sammelbehälter 54 geleitet wird. Die Steuerung 52 ist ferner geeignet, die Schaltventile 40, 46 und 47 anzusteuern und Messwerte der Druckmessvorrichtung 42, der Volumenstrommessvorrichtung 43 und der Differenzdruckmessvorrichtung 44 aufzunehmen und zu verarbeiten.

## Patentansprüche

1. Ophthalmochirurgische Kassette (1), aufweisend:
- einen Kassettenkörper (2), welcher an einer Schmalseite (5) eine erste Aussparung (7) aufweist, welche im Querschnitt in Form eines Kreissegmentes (8) mit einem Kreisbogen (9) ausgebildet ist, wobei die erste Aussparung (7) so ausgebildet ist, dass ein Umfang (94) eines Rades (91) einer Peristaltikpumpe (90) in die erste Aussparung (7) eingreifen kann,
- eine Aspirationsleitung (11) für den Transport eines mittels der Peristaltikpumpe (90) aspirierten Fluides (13),
**dadurch gekennzeichnet, dass**
- entlang des Kreisbogens (9) des Kreissegmentes (8) in radialer Richtung eine zweite Aussparung (14) im Kassettenkörper (2) vorgesehen ist, deren Wandung einen Fluidkanal (15) bildet,
- der Fluidkanal (15) an einem ersten Ende (17) mit der Aspirationsleitung (11) verbunden ist und an einem zweiten Ende (18) mit einer Sammelbehälterzuleitung (16) verbunden ist,
- die Kassette (1) eine Haltevorrichtung (19) zum Halten eines elastischen Abdeckelementes (22) aufweist, mit dem der Fluidkanal (15) abdeckbar ist, wobei die Haltevorrichtung (19) einen konstanten Abstand des Abdeckelementes (22) zur zweiten Aussparung (14) sicherstellt, so dass mittels des auf das Abdeckelement (22) einwirkenden Rades (91) der Peristaltikpumpe (90) Fluid (13) von der Aspirationsleitung (11) durch den abgedeckten Fluidkanal (15) zur Sammelbehälterzuleitung (16) transportierbar ist.

2. Kassette (1) nach Anspruch 1, wobei die Haltevorrichtung (19) das elastische Abdeckelement (22) mittels Formschluss, Kraftschluss oder Stoffschluss mit dem Kassettenkörper (2) hält.

3. Kassette (1) nach Anspruch 1 oder 2, wobei die Haltevorrichtung (19) ein vom Kassettenkörper (2) abstehender Wulst (20) oder eine in den Kassettenkörper (2) eingebrachte Nut (21) ist.

4. Kassette (1) nach Anspruch 3, wobei der Wulst (20) oder die Nut (21) auf einer Fläche (3, 4) senkrecht zur Schmalseite (5) des Kassettenkörpers (2) vorgesehen ist.

5. Kassette (1) nach einem der Ansprüche 1 bis 4, wobei der Querschnitt des Fluidkanals (15) in seiner Längsrichtung variiert.

6. Kassette (1) nach einem der Ansprüche 1 bis 5, wobei sich der Fluidkanal (15) an einem Ende tangential zu seiner Normalen an dem Ende fort erstreckt oder als gekrümmte Bahn (28) fort erstreckt, deren Krümmungsrichtung entgegengesetzt zur Krümmungsrichtung des Kreisbogens (9) des Kreissegmentes (8) der ersten Aussparung (7) ist.

7. Kassette (1) nach einem der Ansprüche 1 bis 6, wobei die Aspirationsleitung (11) und/oder eine in der Kassette (1) vorgesehene Irrigationsleitung (12) eine starre Wandung aus dem gleichen Werkstoff wie der Kassettenkörper (2) besitzt.

8. Kassette (1) nach einem der Ansprüche 1 bis 7, wobei die zweite Aussparung (14) so vorgesehen ist, dass sich das Abdeckelement (22) durch einen auf das Abdeckelement (22) einwirkenden Andrückkörper (92) des Rades (91) der Peristaltikpumpe (90) vollständig in die zweite Aussparung (14) eindrücken lässt.

9. Kassette (1) nach einem der Ansprüche 1 bis 8, wobei die Kassette (1) zwei Hälften aufweist, welche sich zu dem Kassettenkörper (2) zusammenfügen lässt.

10. Ophthalmochirurgisches System (50), welches eine Kassette (1) nach einem der Ansprüche 1 bis 9, eine Peristaltikpumpe (90), eine Steuerung (52) zum Betrieb der Peristaltikpumpe (90) und eine Eingabevorrichtung (53) zum Eingeben von Signalen für die Steuerung (52) aufweist.

## Claims

1. Ophthalmic surgical cassette (1) having:
- a cassette body (2) with, on a narrow side (5), a first recess (7) which is designed in cross section in the form of a circle segment (8) having an arc (9), wherein the first recess (7) is designed such that a periphery (94) of a wheel (91) of a peristaltic pump (90) can engage in the first recess (7),
- an aspiration line (11) for transporting a fluid (13) aspirated by means of the peristaltic pump (90),
**characterized in that**
- a second recess (14) in the cassette body (2) is provided along the arc (9) of the circle segment (8) in the radial direction, the wall of which second recess (14) forms a fluid channel (15),
- the fluid channel (15) is connected at a first end (17) to the aspiration line (11) and is connected at a second end (18) to a collection container supply line (16),
- the cassette (1) has a holding device (19) for holding an elastic cover element (22) with which the fluid channel (15) can be covered, wherein the holding device (19) ensures a constant distance between the cover element (22) and the second recess (14), such that fluid (13) from the aspiration line (11) can be transported through the covered fluid channel (15) to the collection container supply line (16) by means of the wheel (91) of the peristaltic pump (90) acting on the cover element (22).

2. Cassette (1) according to Claim 1, wherein the holding device (19) holds the elastic cover element (22) by means of a form fit, a force fit or a cohesive bond to the cassette body (2).

3. Cassette (1) according to Claim 1 or 2, wherein the holding device (19) is a bead (20) protruding from the cassette body (2), or a groove (21) introduced into the cassette body (2).

4. Cassette (1) according to Claim 3, wherein the bead (20) or the groove (21) is provided on a surface (3, 4) perpendicular to the narrow side (5) of the cassette body (2).

5. Cassette (1) according to one of Claims 1 to 4, wherein the cross section of the fluid channel (15) varies in the longitudinal direction thereof.

6. Cassette (1) according to one of Claims 1 to 5, wherein the fluid channel (15) at one end extends onward tangentially to its normal at the end or extends onward as a curved path (28), of which the direction of curvature is opposite to the direction of curvature of the arc (9) of the circle segment (8) of the first recess (7).

7. Cassette (1) according to one of Claims 1 to 6, wherein the aspiration line (11) and/or an irrigation line (12) provided in the cassette (1) has a rigid wall made from the same material as the cassette body (2).

8. Cassette (1) according to one of Claims 1 to 7, wherein the second recess (14) is provided such that the cover element (22) can be pressed completely into the second recess (14) by a pressure body (92) of the wheel (91) of the peristaltic pump (90) acting on the cover element (22).

9. Cassette (1) according to one of Claims 1 to 8, wherein the cassette (1) has two halves, which can be joined together to form the cassette body (2).

10. Ophthalmic surgical system (50), which has a cassette (1) according to one of Claims 1 to 9, a peristaltic pump (90), a control device (52) for operating the peristaltic pump (90), and an input device (53) for inputting signals for the control device (52).

## Revendications

1. Cassette de chirurgie ophtalmique (1), présentant
- un corps de cassette (2), qui présente, au niveau d'un côté étroit (5), un premier évidement (7) qui est réalisé en section transversale en forme de segment de cercle (8) avec un arc de cercle (9), le premier évidement (7) étant réalisé de telle sorte qu'une périphérie (94) d'une roue (91) d'une pompe péristaltique (90) puisse s'engager dans le premier évidement (7),
- une conduite d'aspiration (11) pour le transport d'un fluide (13) aspiré au moyen de la pompe péristaltique (90),
**caractérisée en ce que**
- un deuxième évidement (14) est prévu dans le corps de cassette (2) le long de l'arc de cercle (9) du segment de cercle (8) dans la direction radiale, sa paroi formant un canal fluidique (15),
- le canal fluidique (15) est connecté au niveau d'une première extrémité (17) à la conduite d'aspiration (11) et est connecté au niveau d'une deuxième extrémité (18) à une conduite d'amenée d'un récipient collecteur (16),
- la cassette (1) présente un dispositif de retenue (19) pour retenir un élément de recouvrement élastique (22), avec lequel le canal fluidique (15) peut être recouvert, le dispositif de retenue (19) maintenant un espacement constant entre l'élément de recouvrement (22) et le deuxième évidement (14), de telle sorte qu'au moyen de la roue (91) de la pompe péristaltique (90) agissant sur l'élément de recouvrement (22), du fluide (13) puisse être transporté depuis la conduite d'aspiration (11) à travers le canal fluidique recouvert (15) jusqu'à la conduite d'amenée du récipient collecteur (16).

2. Cassette (1) selon la revendication 1, dans laquelle le dispositif de retenue (19) retient l'élément de recouvrement élastique (22) au moyen d'un engagement par correspondance géométrique, par force ou par liaison de matière avec le corps de cassette (2).

3. Cassette (1) selon la revendication 1 ou 2, dans lequel le dispositif de retenue (19) est un bourrelet (20) saillant depuis le corps de cassette (2) ou une rainure (21) pratiquée dans le corps de cassette (2).

4. Cassette (1) selon la revendication 3, dans laquelle le bourrelet (20) ou la rainure (21) est prévu(e) sur une surface (3, 4) perpendiculairement au côté étroit (5) du corps de cassette (2).

5. Cassette (1) selon l'une quelconque des revendications 1 à 4, dans laquelle la section transversale du canal fluidique (15) varie dans sa direction longitudinale.

6. Cassette (1) selon l'une quelconque des revendications 1 à 5, dans laquelle le canal fluidique (15) continue de s'étendre au niveau d'une extrémité tangentiellement à sa normale à l'extrémité ou continue de s'étendre sous forme de trajet courbe (28) dont la direction de courbure est opposée à la direction de courbure de l'arc de cercle (9) du segment de cercle (8) du premier évidement (7).

7. Cassette (1) selon l'une quelconque des revendications 1 à 6, dans laquelle la conduite d'aspiration (11) et/ou une conduite d'irrigation (12) prévue dans la cassette (1) possède (nt) une paroi rigide en le même matériau que le corps de cassette (2).

8. Cassette (1) selon l'une quelconque des revendications 1 à 7, dans laquelle le deuxième évidement (14) est prévu de telle sorte que l'élément de recouvrement (22) puisse pénétrer complètement dans le deuxième évidement (14) à travers un corps de pression (92) de la roue (91) de la pompe péristaltique (90) agissant sur l'élément de recouvrement (22).

9. Cassette (1) selon l'une quelconque des revendications 1 à 8, dans laquelle la cassette (1) présente deux moitiés qui peuvent être assemblées pour former le corps de cassette (2).

10. Système de chirurgie ophtalmique (50) qui présente une cassette (1) selon l'une quelconque des revendications 1 à 9, une pompe péristaltique (90), une commande (52) pour le fonctionnement de la pompe péristaltique (90) et un dispositif de saisie (53) pour saisir des signaux pour la commande (52).
